(19) Europäisches Patentamt
European Patent Office
Office européen des brevets

(11) Publication number: **0 218 736 B1**

(12) # EUROPEAN PATENT SPECIFICATION

(45) Date of publication of patent specification: **07.04.93**

(51) Int. Cl.$^5$: **C07D 409/06**, C07D 409/14, A01N 43/50

(21) Application number: **85112797.7**

(22) Date of filing: **09.10.85**

The file contains technical information submitted after the application was filed and not included in this specification

(54) **A ketene S,S-acetal derivative, a process for manufacturing thereof and a method for curing mycosis by administering it.**

(43) Date of publication of application:
**22.04.87 Bulletin 87/17**

(45) Publication of the grant of the patent:
**07.04.93 Bulletin 93/14**

(84) Designated Contracting States:
**AT BE CH DE FR GB IT LI LU NL SE**

(56) References cited:
**EP-A- 0 061 794**
**EP-A- 0 080 103**
**DD-A- 202 713**
**DE-A- 2 134 499**

(73) Proprietor: **NIHON NOHYAKU CO., LTD.**
**1-2-5, Nihonbashi**
**Chuo-ku Tokyo(JP)**

(72) Inventor: **Soe, Akira**
**6-2-312, Saiwaicho**
**Yamatotakada-shi(JP)**
Inventor: **Kanno, Hideo**
**2-2-708, Shirakawa-3-chome**
**Ibaraki-shi(JP)**
Inventor: **Hasegawa, Nobu**
**1-10, Uedanishimachi**
**Nishinomiya-shi(JP)**
Inventor: **Miyagi, Yukio**
**7-23-816, Nankonaka-4-chome**
**Suminoe-ku Osaka(JP)**
Inventor: **Nishimura, Akira**
**5-6, Hondacho**
**Kawachinagano-shi(JP)**
Inventor: **Konaka, Shigeo**
**9-4-213, Takawashi-4-chome**
**Habikino-shi(JP)**
Inventor: **Ohmi, Tetsuto**
**1-28, Nishinoyamacho**
**Kawachinagano-shi(JP)**
Inventor: **Munechika, Yukimi**
**3-8-1, Toge**
**Hashimoto-shi(JP)**
Inventor: **Uchida, Matazaemon**
**14-22, Daishicho**
**Kawachinagano-shi(JP)**
Inventor: **Ikeda, Kenichi**
**13-13, Tsuganodai-1-chome**
**Chiba-shi(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.5x/3.0.1)

(74) Representative: **Patentanwälte Grünecker, Kinkeldey, Stockmair & Partner**
**Maximilianstrasse 58**
**W-8000 München 22 (DE)**

## Description

This invention relates to ketene S,S-acetal derivatives useful as antimycotic agents and agricultural chemicals.

EP-A-61794 discloses antimicrobial imidazole derivatives and EP-A-80103 discloses azolylvinyldithioacetals.

The present invention relates to ketene S,S-acetal derivatives represented by the general formula (I):

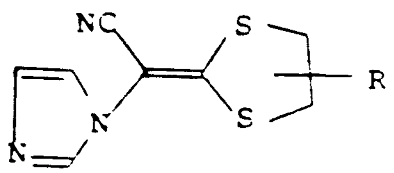

(I)

wherein R represents a hydrogen atom; an alkyl group having 1 to 8 carbon atom; a cycloalkyl group having 3 to 6 carbon atoms; a methylene group; a lower alkenyl group; a lower alkyl group substituted by a halogen atom, a cyano group, a lower alkoxyl group, a lower alkylthio group, a carbamoyl group, an acyl group, or an alkenoyloxy group; a phenyl group represented by

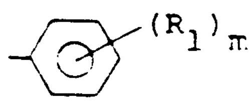

(in which $R_1$ represents a hydrogen atom, a halogen atom, a straight or branched chain lower alkyl group, a lower alkoxyl group which may be substituted by one or more halogen atoms, a phenoxy group or a methylenedioxy group, and m represents an integer of 1 to 3); a benzyl group; a methylenedioxybenzyl group; a phenoxyalkyl group; a phenoxyalkyl group substituted by a halogen atom; a naphthyl group; or a substituted or unsubstituted pyridyl group.

The compounds represented by the general formula (I) are useful as antimycotic agents and agricultural chemicals; in particular, not only fungicides and plant growth regulators but also insecticides.

The substituent R in the above general formula (I) includes a hydrogen atom; straight or branched chain alkyl groups such as methyl, ethyl, n-propyl, i-propyl, n-butyl, i-butyl, s-butyl, t-butyl, n-pentyl, i-pentyl, neopentyl, n-hexyl or n-octyl; cycloalkyl groups such as cyclopropyl or cyclohexyl; a methylene group; lower alkenyl groups such as vinyl or allyl; lower alkyl groups substituted by a halogen atom such as chloromethyl or dichloroethyl; lower alkyl groups substituted by a cyano group such as cyanomethyl; lower alkyl groups substituted by a lower alkoxyl such as methoxymethyl or methoxyethyl or a lower alkylthio group such as methylthiomethyl; lower alkyl groups substituted by a carbamoyl group such as carbamoylmethyl; lower alkyl groups substituted by an acyl group; lower alkyl groups substituted by an alkenoyloxy groups such as acryloylmethyl; phenyl groups represented by

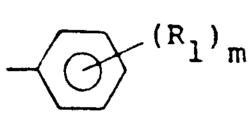

(in which $R_1$ represents a hydrogen atom, a halogen atom, a straight or branched chain lower alkyl group, a lower alkoxyl group which may be substituted by one or more halogen atoms, a phenoxy group or a methylenedioxy group, and m represents an integer of 1 to 3); a benzyl group; a methylenedioxybenzyl group; a phenoxyalkyl group such as phenoxymethyl; a phenoxyalkyl group substituted by a halogen atom; a naphthyl group; or a substituted or unsubstituted pyridyl group.

The compound represented by the general formula (I) of this invention can be synthesized, for example, by the process shown below:

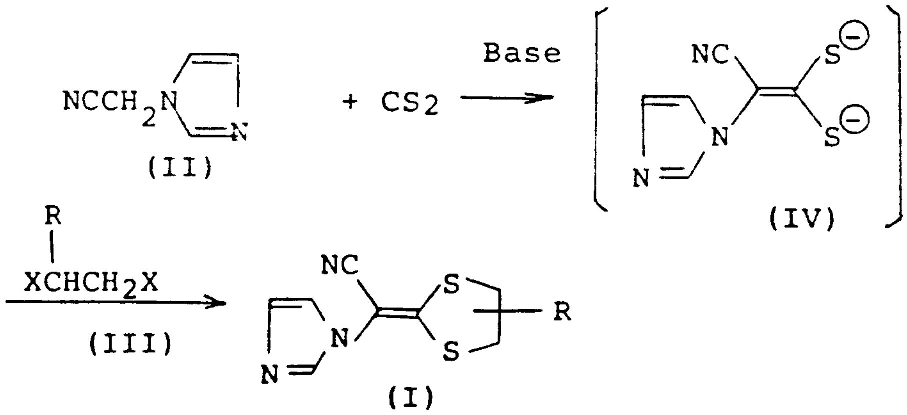


(II) (III) (IV) (I)

wherein R is as defined above, and X represents a halogen atom, a mesyloxy or a tosyloxy group. The compound represented by the general formula (I) can be obtained by reacting 1-cyanomethylimidazole represented by the structural formula (II) with carbon disulfide in the presence of a base and a solvent to form an intermediate represented by the structural formula (IV), and reacting the intermediate with a compound represented by the general formula (III) without isolating same.

As the solvent usable in the invention, any solvent may be used as long as it does not inhibit the progress of the reaction, and there can be exemplified, for example, alcohols such as methanol, ethanol or isopropanol, dimethylsulfoxide, dimethylformamide, hexamethylenephosphoroamide or water. These solvents can be used alone or as a mixture thereof.

As the base usable in this invention, there can be exemplified sodium carbonate, potassium carbonate, sodium hydrogencarbonate, potassium hydrogencarbonate, sodium hydroxide, potassium hydroxide and potassium t-butoxide. These can be used in solid state or in solution.

Although it is sufficient that the reaction temperature is selected in the range of 0° to 100°C, it is particularly preferable to carry out the reaction at a temperature near room temperature.

It is sufficient that the reaction time is properly selected in the range of 0.5 to 24 h.

It is sufficient that the amount of the base used is selected in the range of 2 to 4 moles per mole of 1-cyanomethylimidazole represented by the structural formula (IV).

It is sufficient that after completion of the reaction, the reaction solution is treated in the usual way. For example, the reaction product is extracted and separated with a suitable solvent and can then be purified by recrystallization or column chromatography.

The compound represented by the general formula (I) is often obtained as a mixture of two kinds of the geometrical isomers shown below.

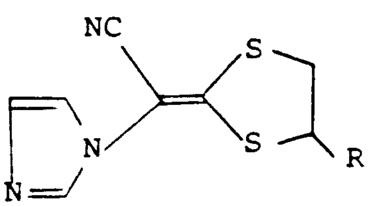

(Z isomer)

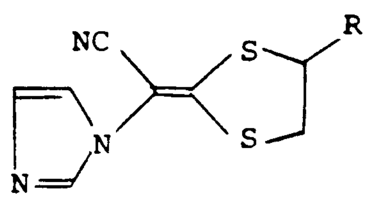

(E isomer)

The aforesaid mixture of Z and E isomers can often be isolated into the two isomers by a suitable separation method, for example, recrystallization or chromatography.

This invention includes the geometrical isomers, i.e., the E and Z isomers, and all mixtures of the two isomers in any ratio.

Typical examples of the compounds represented by the general formula (I) are shown in Table 1.

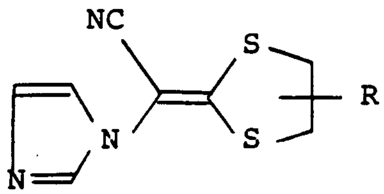

(I)

Table 1

| Compound No. | R | Physical property, melting point, refractive index, NMR value (TMS/$CDCl_3$) |
|---|---|---|
| 1 | H | Melting point 126.5°C |
| 2 | $CH_3$ | Melting point 97.6°C |
| 3 | $C_2H_5$ | $n_D^{17}$ 1.6246 |
| 4 | n-$C_3H_7$ | $n_D^{27}$ 1.6065 |
| 5 | i-$C_3H_7$ | Melting point 86.7°C (Z isomer) |
| 6 | i-$C_3H_7$ | Melting point 55.8°C (E isomer) |
| 7 | n-$C_4H_9$ | $n_D^{27}$ 1.5830 |
| 8 | i-$C_4H_9$ | Melting point 73.3°C (Z isomer) |
| 9 | i-$C_4H_9$ | Melting point 118.1°C (F isomer) |

| Compound No. | R | Physical property, melting point, refractive index, NMR value (TMS/$CDCl_3$) |
| --- | --- | --- |
| 10 | s-$C_4H_9$ | $n_D^{16}$ 1.6011 (Z isomer) |
| 11 | s-$C_4H_9$ | $n_D^{16}$ 1.6089 (E isomer) |
| 12 | t-$C_4H_9$ | Melting point 151.7°C |
| 13 | n-$C_5H_{11}$ | $n_D^{16.5}$ 1.5931 (Z isomer) |
| 14 | n-$C_5H_{11}$ | $n_D^{16.5}$ 1.5949 (E isomer) |
| 15 | i-$C_5H_{11}$ | Melting point 74.3°C (Z isomer) |
| 16 | i-$C_5H_{11}$ | Melting point 111.4°C (E isomer) |
| 17 | neo-$C_5H_{11}$ | Melting point 96.2°C (Z isomer) |
| 18 | neo-$C_5H_{11}$ | Melting point 107.7°C (E isomer) |
| 19 | n-$C_6H_{13}$ | $n_D^{17}$ 1.5908 (Z isomer) |
| 20 | n-$C_6H_{13}$ | Melting point 48.2°C (E isomer) |
| 21 | 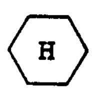 | Melting point 113.9°C (Z isomer) |
| 22 | 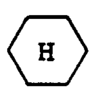 | Melting point 103.8°C (E isomer) |
| 23 | =$CH_2$ | Melting point 115.7°C |
| 24 | -CH=$CH_2$ | $n_D^{27}$ 1.6422 |

| Compound No. | R | Physical property, melting point, refractive index, NMR value (TMS/$CDCl_3$) |
|---|---|---|
| 25 | $-CH_2Cl$ | Melting point 96.2°C |
| 26 | $-CHClCH_2Cl$ | $n_D^{16}$ 1.6103 |
| 27 | $-CH_2CN$ | $n_D^{18}$ 1.6167 |
| 28 | $-CH_2CH_2OCH_2CH_3$ | $n_D^{24}$ 1.6009 |
| 29 | $-CH_2SCH_3$ | $n_D^{13.5}$ 1.6412 |
| 30 | $-CH_2CONH_2$ | Viscous oily substance 3.64 (m, 2H), 4.20 (m, 3H), 5.27 (br, 2H) 6.96, 7.05, 7.52 (1H on hetero ring for each) |
| 31 | $-CH_2CH_2COCH_3$ | $n_D^{18}$ 1.6086 |
| 32 | $-CH_2O_2CCH=CH_2$ | $n_D^{18}$ 1.6082 |
| 33 | 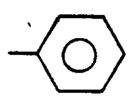 | $n_D^{24}$ 1.6417 |
| 34 | 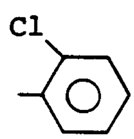 | Melting point 119.4°C (Z isomer) |
| 35 | 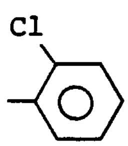 | Melting point 141.5°C (E isomer) |
| 36 | 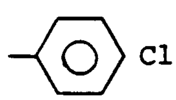 | $n_D^{24}$ 1.6083 |

| No. | R | Physical property, melting point, refractive index, NMR value (TMS/CDCL$_3$) |
|---|---|---|
| 37 | 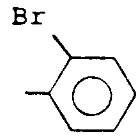 | Viscous oily substance (Z isomer)<br>3.54-4.20 (m, 2H), 5.69 (DD, 1H) 7.00-7.75 (m, 7H) |
| 38 | 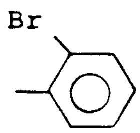 | Viscous oily substance (E isomer)<br>3.45-4.10 (m, 2H), 5.76 (dd, 1H) 7.00-7.85 (m, 7H) |
| 39 | 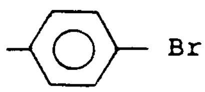 | Viscous oily substance (z isomer)<br>3.6-4.0 (m, 2H), 5.22 (dd, 1H) 6.9-7.81 (m, 7H) |
| 40 | 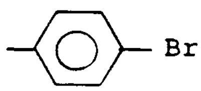 | Melting point 148.8°C (E isomer) |
| 41 | 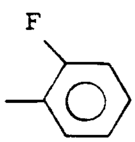 | Viscous oily substance (Z isomer) |
| 42 | 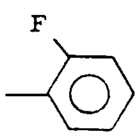 | Melting point 119°C (E isomer) |
| 43 | 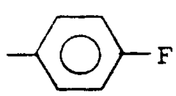 | Viscous oily substance<br>3.6-3.9 (m, 2H), 5.1-5.5 (m, 1H) 6.9-7.8 (m, 7H) |
| 44 | 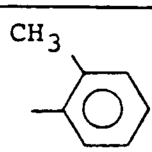 | $n_D^{18}$ 1.6313 (Z isomer) |

| Compound No. | R | Physical property, melting point, refractive index, NMR value ($TMS/CDCl_3$) |
|---|---|---|
| 45 | 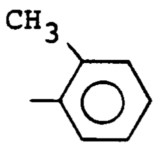 | Melting point 123.3°C (E isomer) |
| 46 | 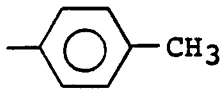 | $n_D^{24}$ 1.6360 |
| 47 | 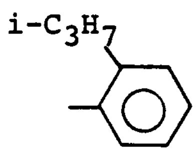 | $n_D^{16}$ 1.6196 |
| 48 | 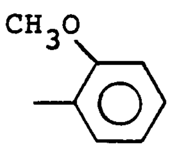 | Viscous oily substance (Z isomer) |
| 49 | 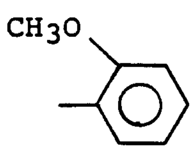 | Viscous oily substance (E isomer)<br>3.71 (d, 2H), 3.82 (s, 3H), 5.68 (t, 1H)<br>6.87, 7.00, 6.72 (1H for each, H on azole ring)<br>7.00-7.60 (m, 4H) |
| 50 | 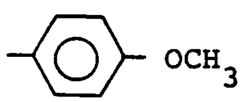 | Viscous oily substance (Z isomer)<br>3.75 (d, 2H), 3.77 (s, 3H), 5.17 (t, 1H), 6.74-7.60 (m, 7H) |
| 51 | 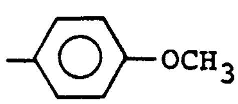 | Viscous oily substance (E isomer)<br>3.67 (d, 2H), 3.80 (s, 3H), 5.24 (t, 1H), 6.70-7.65 (m, 7H) |

| Compound No. | R | Physical property, melting point, refractive index, NMR value (TMS/$CDCl_3$) |
|---|---|---|
| 52 | 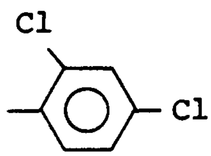 | Melting point 110.5°C (Z isomer) |
| 53 | 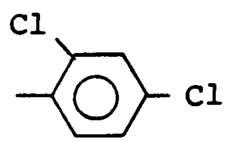 | Melting point 100.4°C (E isomer) |
| 54 | 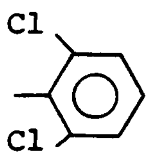 | Viscous oily substance (Z isomer) |
| 55 | 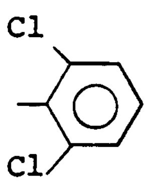 | Viscous oily substance (E isomer)<br>3.42 (dd, 1H), 4.43 (t, 1H), 6.16 (dd, 1H), 7.18-7.40 (m, 3H), 6.98, 7.08, 7.56 (1H for each, H on azole ring) |
| 56 | 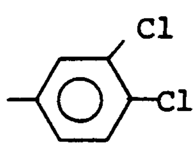 | Viscous oily substance<br>3.6-3.9 (m, 2H), 5.0-5.4 (m, 1H), 6.9-7.8 (m, 6H) |
| 57 | 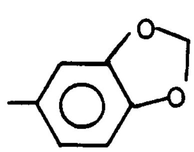 | Viscous oily substance<br>3.64, 3.72 (d, 2H for each), 5.11, 5.19 (t, 1H for each), 5.92, 5.95 (s, 2H for each), 6.60-6.95 (m, 3H), 7.00, 7.08, 7.53 (1H for each, H on azole ring) |

| Compound No. | R | Physical property, melting point, refractive index, NMR value (TMS/$CDCl_3$) |
|---|---|---|
| 58 | 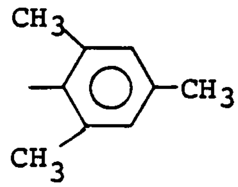 | Melting point 187.5°C (Z isomer) |
| 59 | 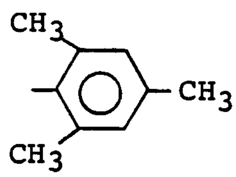 | Melting point 214.0°C (E isomer) |
| 60 | 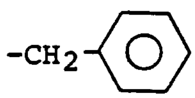 | Melting point 102.7°C |
| 61 | 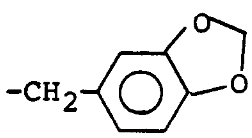 | $n_D^{17}$ 1.6258 |
| 62 | 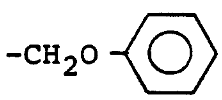 | $n_D^{18}$ 1.6315 |
| 63 | 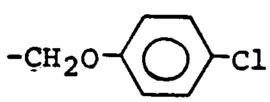 | $n_D^{18}$ 1.6213 |
| 64 | 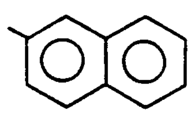 | Melting point 135.0°C |
| 65 | 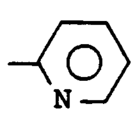 | Viscous oily substance<br>3.65-4.38 (m, 2H), 5.22-5.60 (m, 1H), 7.00-7.95 (m, 6H), 8.54-8.76 (m, 1H) |

| Compound No. | R | Physical property, melting point, refractive index, NMR value (TMS/$CDCl_3$) |
|---|---|---|
| 66 | 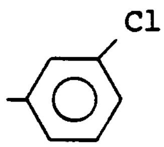 | Viscous oily substance<br>3.75 (m, 2H), 5.20 (m, 1H), 6.80-7.80 (m, 7H) |
| 67 | 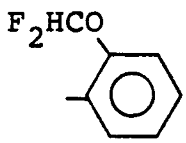 | $n_D^{18.5}$ 1.6063 |
| 68 | 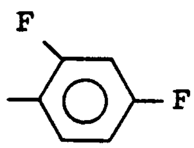 | Viscous oily substance (Z isomer)<br>3.83 (m, 2H), 5.46 (dd, 1H) 6.70-7.80 (m, 6H) |
| 69 | 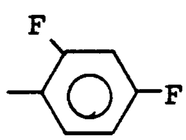 | Viscous oily substance (E isomer)<br>3.75 (m, 2H), 5.52 (dd, 1H), 6.60-7.80 (m, 6H) |
| 70 | 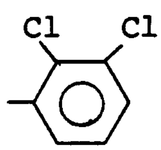 | $n_D^{17.5}$ 1.6358 (E isomer) |
| 71 | 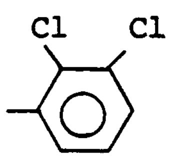 | $n_D^{17.5}$ 1.6458 (Z isomer) |
| 72 | 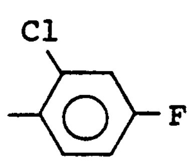 | Viscous oily substance (Z isomer)<br>3.83 (m, 2H), 5.59 (dd, 1H), 6.75-7.70 (m, 6H) |

| Compound No. | R | Physical property, melting point, refractive index, NMR value (TMS/$CDCl_3$) |
|---|---|---|
| 73 | 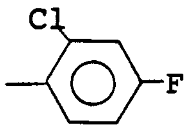 | m.p. 146-152°C (E isomer) |
| 74 | 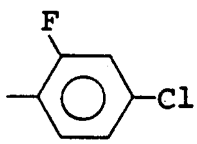 | Viscous oily substance (Z isomer)<br>3.38 (m, 2H), 5.42 (dd, 1H), 6.90-7.70 (m, 6H) |
| 75 | 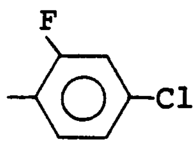 | m.p. 114-117°C (E isomer) |
| 76 | 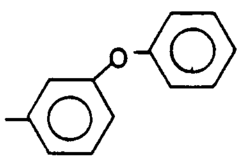 | $n_D^{22.0}$ 1.6401 |
| 77 | 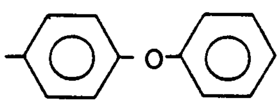 | $n_D^{22.0}$ 1.6262 |
| 78 | 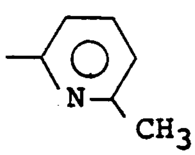 | $n_D^{25.5}$ 1.6432 |
| 79 | 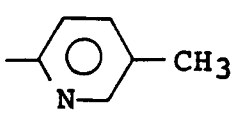 | $n_D^{25.5}$ 1.6332 |

Preferred compounds are those wherein R represents a lower alkyl group, a lower alkyl group substituted by a lower alkoxyl or alkylthio group, or

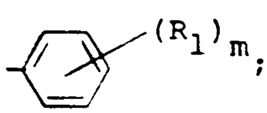

in which $R_1$ represents a halogen atom, especially chlorine or bromine atom, or a straight or branched chain lower alkyl group, especially methyl, and m represents an integer of 1 to 3.

Examples of this invention are shown below.

Example 1

Synthesis of 2-(1-imidazolyl)-2-(4-isobutyl-1,3-dithiolan-2-ylidene)acetonitrile (Compound Nos. 8 and 9)

To a mixed solution of 0.55 g (0.005 mole) of 1-cyanomethylimidazole, 0.4 g (0.005 mole) of carbon disulfide and 10 ml of dimethyl sulfoxide was added 0.8 g (0.014 mole) of potassium hydroxide powder with stirring, and the reaction was carried out at room temperature for 1 h. Then, 1.5 g (0.006 mole) of 1,2-dibromo-4-methylpentane was added dropwise with stirring, and the resulting solution was subjected to reaction for 2 h. After completion of the reaction, 20 ml of water was added to the reaction solution, after which the resulting mixture was subjected to extraction with ethyl acetate, and the organic layer was washed with water and dried. The solvent was distilled off and the residue was purified by silica gel chromatography to obtain 0.45 g of the Z isomer and 0.3 g of the E isomer individually in the form of colorless crystals.

The Z isomer (Compound No. 8): melting point 73.3°C, yield 34%.

The E isomer (Compound No. 9): melting point 118.1°C, yield 23%.

Example 2

Synthesis of 2-(1-imidazolyl)-2-(4-chloromethyl-1,3-dithiolan-2-ylidene)acetonitrile (Compound No. 25).

To a mixed solution of 0.55 g (0.005 mole) of 1-cyanomethylimidazole, 0.4g (0.005 mole) of carbon disulfide and 10 ml of dimethyl sulfoxide was added 0.8 g (0.014 mole) of potassium hydroxide powder with stirring, and the reaction was carried out at room temperature for 1 h. Then, 1.4 g (0.006 mole) of 1,2-dibromo-3-chloropropane was added dropwise with stirring, and the resulting solution was subjected to reaction for 2 h. After completion of the reaction, 20 ml of water was added to the reaction solution, after which the resulting mixture was subjected to extraction with ethyl acetate, and the organic layer was washed with water and dried. The solvent was distilled off and the residue was purified by silica gel chromatography and recrystallized from ethyl acetate-n-hexane to obtain 0.8 g of the desired compound in the form of colorless crystals: melting point 96.2°C, yield 62%.

Example 3

Synthesis of 2-(1-imidazolyl)-2-(4-methylidene-1,3-dithiolan-2-ylidene)acetonitrile (Compound No. 23)

In 10 ml of tetrahydrofuran were dissolved 0.52 g (0.002 mole) of the 2-(1-imidazolyl)-2-(4-chloromethyl-1,3-dithiolan-2-ylidene)acetonitrile obtained in Example 2 and 0.31 g of 1,8-diazabicyclo-[5,4,0]-7-undecene, and the reaction was carried out with heating under reflux for 1 h. After the reaction solution was allowed to cool, the deposited salt was separated by filtration and the filtrate was concentrated to obtain crude crystals, which were then recrystallized from ethyl acetate-n-hexane to obtain 0.35 g of the desired compound in the form of crystals: melting point 115.7°C, yield 79%.

Example 4

Synthesis of 2-(1-imidazolyl)-2-[4-(2,4-dichlorophenyl)-1,3-dithiolan-2-ylidene]acetonitrile (Compound Nos. 52 and 53)

To a mixed solution of 0.55 g (0.005 mole) of 1-cyanomethylimidazole, 0.4 g (0.005 mole) of carbon disulfide and 10 ml of dimethyl sulfoxide was added 0.8 g (0.014 mole) of potassium hydroxide powder with stirring, and the reaction was carried out at room temperature for 1 h. Then, 2.0 g (0.006 mole) of 2',4'-dichloro-1,2-dibromoethylbenzene was added dropwise with stirring, and the resulting solution was subjected to reaction for 2 h. After completion of the reaction, 20 ml of water was added to the reaction solution, and the resulting mixture was subjected to extraction with ethyl acetate, and the organic layer was washed with water and dried. The solvent was distilled off and the residue was purified by silica gel chromatography to obtain 0.25 g of the Z isomer and 0.5 g of the E isomer individually in the form of a

yellow viscous substance: yield (total yield of the Z and E isomers) 42%, melting points 110.5°C (the Z isomer) and 100.4°C (the E isomer).

Example 5

Synthesis of 2-(1-imidazolyl)-2-[4-(2-isopropylphenyl)-1,3-dithiolan-2-ylidene]acetonitrile (Compound No. 47)

To a mixed solution of 0.55 g (0.005 mole) of 1-cyanomethylimidazole, 0.4 (0.005 mole) of carbon disulfide and 10 ml of dimethylformamide was added 0.8 g (0.014 mole) of potassium hydroxide powder, and the reaction was carried out with stirring at room temperature for 1 h. Then, 1.8 g (0.006 mole) of 2'-isopropyl-1,2-dibromoethylbenzene was added dropwise with stirring, and the resulting solution was subjected to reaction for another 2 h. After completion of the reaction, 20 ml of water was addd to the reaction solution, and the resulting mixture was subjected to extraction with ethyl acetate, and the organic layer was washed with water and dried. The solvent was distilled off and the residue was purified by silica gel chromatography to obtain the desired compound in the form of a light-yellow oily substance: $n_D^{16}$ 1.6196, yield 34%.

Example 6

Synthesis of 2-(1-imidazolyl)-2-[4-(2-chlorophenyl)-1,3-dithiolan-2-ylidene]acetonitrile (Compound Nos. 34 and 35)

To a mixed solution of 2.2 g (0.02 mole) of 1-cyanomethylimidazole, 1.60 g (0.02 mole) of carbon disulfide and 10 ml of dimethyl sulfoxide was added 3.0 g (0.05 mole) of potassium hydroxide powder, and the reaction was carried out with stirring at room temperature for 1 h. Then, 4.2 g (0.02 mole) of 2'-chloro-(1,2-dichloroethyl)benzene was added dropwise with stirring, and the resulting solution was subjected to reaction for another 2 h. After completion of the reaction, 20 ml of water was added to the reaction solution, after which the resulting mixture was subjected to extraction with ethyl acetate, and the organic layer was washed with water and dried. The solvent was distilled off and the residue was purified by silica gel chromatography to obtain 2.0 g of the Z isomer and 2.4 g of the E isomer individually in the form of light-yellow crystals.

The Z isomer: melting point 119.4°C, yield 31%.

The E isomer: melting point 141.5°C, yield 38%.

Example 7

Synthesis of 2-(1-imidazolyl)-2-[4-(2-chlorophenyl)-1,3-dithiolan-2-ylidene]acetonitrile (Compound Nos. 34 and 35)

To a mixed solution of 2.2 g (0.02 mole) of 1-cyanomethylimidazole, 1.60 g (0.02 mole) of carbon disulfide and 10 ml of dimethyl sulfoxide was added 3.0 g (0.05 mole) of potassium hydroxide powder, and the reaction was carried out with stirring at room temperature for 1 h. Then, 6.0 g (0.02 mole) of 2'-chloro-(1,2-dibromoethyl)benzene was added dropwise with stirring, and the resulting solution was subjected to reaction for another 2 h. After completion of the reaction, 20 ml of water was added to the reaction solution, after which the resulting mixture was subjected to extraction with ethyl acetate, and the organic layer was washed with water and dried. The solvent was distilled off and the residue was purified by silia gel chromatography to obtain 0.3 g of the Z isomer and 1.3 g of the E isomer individually in the form of light-yellow crystals.

The Z isomer: melting point 119.4°C, yield 5%.

The E isomer: melting point 141.5°C, yield 20%.

Example 8

Synthesis of 2-(1-imidazolyl)-2-[4-(2-chlorophenyl)-1,3-dithiolan-2-ylidene]acetonitrile (Compound Nos. 34 and 35)

To a mixed solution of 2.2 g (0.02 mole) of 1-cyanomethylimidazole, 1.60 g (0.02 mole) of carbon disulfide and 10 ml of dimethyl sulfoxide was added 3.0 g (0.05 mole) of potassium hydroxide powder, and

the reaction was carried out with stirring at room temperature for 1 h. Then, 6.6 g (0.02 mole) of 2'-chloro-(1,2-dimesyloxyethyl)benzene was added dropwise with stirring, and the resulting solution was subjected to reaction for another 2 h. After completion of the reaction, 20 ml of water was added to the reaction solution, after which the resulting mixture was subjected to extraction with ethyl acetate, and the organic layer was washed with water and dried. The solvent was distilled off and the residue was purified by silica gel chromatography to obtain 1.3 g of the Z isomer and 1.8 g of the E isomer individually in the form of light-yellow crystals.

The Z isomer: melting point 119.4°C, yield 20%.

The E isomer: melting point 141.5°C, yield 28%.

The compounds of this invention are antimycotic agents useful for preventing fungous infection of human beings and animals. For example, these compounds can be used for curing mycoses such as local mycotic infection, mucosal mycotic infection or systemic mycotic infection which are caused by Dermatophytes such as Microsporum, Epidermophyton or Trichophyton and Candida.

The compounds of this invention can be mixed with conventional chemotherapeutically acceptable diluents or carriers and if desired, other excipients, and can be used in pharmaceutical forms such as solutions, creams, suppositories, ointments or tablets.

When used as antimycotic agents, the compounds of this invention can be used as local liniments in pharmaceutical forms such as creams, ointments or solution. When they are used in the form of an endermic solution, their practical concentration is considered to be suitably 0.1% or more.

The present drugs may be used, of course, in an admixture with other antibacterial agents such as amphotericin B, nystatin, trichomycin, variotin or clotrimazole.

Further, the compounds of this invention are useful as agricultural and horticultural fungicides. For example, they are very effective against various phytopathogenic diseases, e.g., rice blast (Piricularia oryzae); powdery mildew of barley and wheat (Erysiphe graminis), and other powdery mildews of various host plants such as that of cucumber (Sphaerotheca fulginea), that of apple (Podosphaera leucotricha) and that of grape (Uncinula necator); rust of wheat (Puccinia vecondita); Crown rust of oats (Puccinia coronate) and rust of other host plants: late blight of tomato (phytophthora capsici) and phytophthora rot of other host plants.

When the compound of this invention is used as active ingredient in an agricultural and a horticultural fungicide, the fungicide is prepared into a formulation suitable for use in a conventional manner as an agricultural chemical. For example, the fungicide is prepared in the form of dust, granules, fine granules, wettable powder, emulsifiable concentrate, oily solution, aerosol, floating dust, fumigants, preparations suitable for vaporizing the active ingredient by heat or other physical means or tablets by mixing the compound of the invention with adjuvants, and is applied to stalks and leaves of vegetables, flowering plants, crops for industrial use, fruit trees and other trees as it is or after diluted to a suitable volume with water. Although in this invention the dosage of the active ingredient varies depending on the kind of the compound, plant to be treated or the way of using, it can be selected in the range of 5 to 500 g per 10 ares.

When applied, the compound of this invention can also be used in combination with other agricultural chemicals, fertilizers or plant nutrients which can be used similarly to the compound.

For example, when a phytopathogenic disease is controlled by using an agricultural and horticultural fungicide comprising the compound of this invention as active ingredient, the fungicide can be made into a multiple-purpose preventing and curing agent by mixing therewith an agent for preventing and curing other diseases and/or vermin insects which break out simultaneously with the aforesaid disease.

Some Test Examples and Formulation Examples are given below in order to demonstrate the usefulness of the compounds of this invention.

Test Example 1

Test for antifungal activity against Trichophytonmentagrophytes

In 1 liter of water were dissolved 10 g of peptone and 4.0 g of glucose and the resulting solution was adjusted to pH 6.0, after which a drug containing 50 ppb (1% DMSO solution) of each active ingredient was added. Into a 3.5 $\phi$ cm Petri dish was poured 30 ml of the thus obtained Sabouraud medium to prepare an agar plate. Then, the inoculum of the fungus precultured was poured onto the plate in an amount of 0.1 ml per Petri dish. The thus prepared plates were incubated at 28°C for 4-6 days. The test was carried out in duplication for each compound and the results per dish evaluated macroscopically are shown in Table 2.

Evaluation criterion

| | |
|---|---|
| + | Multiplication of cells was completely inhibited. |
| + | Multiplication of cells was inhibited. |
| ++ | White colonies were formed. |
| +++ | The diameter of white colonied increased. |

Table 2

| Compound No. | Degree of growth | |
|---|---|---|
| 4 | ± | + |
| 28 | ± | + |
| 33 | ± | ± |
| 35 | ± | + |
| 36 | ± | ± |
| 37 | ± | ± |
| 38 | + | + |
| 39 | ± | ± |
| 40 | ± | ± |
| 41 | + | ++ |
| 42 | + | ± |
| 43 | ± | ± |
| 46 | ± | ± |
| 52 | ± | ± |
| 53 | ± | ± |
| 59 | ± | ± |
| 64 | + | + |
| 65 | + | + |
| Control (no compound added) | | |

Test Example 2

Curing effect test against trichophytosis which was artificially infected in guinea pigs

Hartley strain white male guinea pigs (400 to 600 g) were used as test animals. The hair in three spots on the back of each guinea pig was sheared and then removed in a circle having a diameter of about 3 cm by use of depilatory cream, after which the skin in the depilated spots was lightly rubbed with sandpaper. The thus treated spots were inoculated with 0.1 ml ($10^6$ spores/spot) of culture of Trichophyton mentagrophytes IFO-5466 strain grown on Sabouraud glucose agar media. Each test drug prepared by using

polyethylene glycol 300 as base was applied to the inoculation spots once daily for 11 consecutive days in an amount of 0.2 ml per inoculation spot, starting 72 h after the inoculation. The evaluation was conducted by macroscopical judgement and reverse culture test.

a) Macroscopical judgement:

After the inoculation, the alleviation and development of symptoms in the above-mentioned spots were observed every day for 15 days. The results are shown in Table 3.

0: No symptom was observed.
1: A few small erythemas were observed.
2: Erythemas were insularly scattered or fused into one, and rubefaction was observed around them.
3: Scales were observed, and then formation of a thick lesion was observed.
4: Lesion reached an extreme stage with bleeding.

The results obtained are shown in Table 3.

b) Reverse culture test

Each guinea pig was sacrified 15 days after the inoculation, after which the skin in the whole inoculation spots was cut off and the skin cut-off was further cut into small pieces (about 5 mm square). The pieces thus prepared were placed on Sabouraud glucose agar medium containing 20 i.u./ml of penicillin G and 40 µg/ml of streptomycin, and then cultured at 27°C for 2 weeks. The evaluation was carried out by investigating the colonies. The results obtained are shown in Table 4.

Table 3 (Macroscopical judgement)

| Drug tested (Concentration) \ Days | | 4 | 5 | 6 | 7 | 8 | 9 | 10 | 11 | 12 | 13 | 14 | 15 |
|---|---|---|---|---|---|---|---|---|---|---|---|---|---|
| Compound 53 | (1%) | 0.5 | 1 | 1 | 1 | 1 | 1 | 1 | 1.5 | 1.5 | 1.5 | 2.5 | 2 |
| | (0.1%) | 1 | 2 | 2 | 2 | 2.5 | 3 | 3 | 3.5 | 3.5 | 3.5 | 3.5 | 3.5 |
| Compound 35 | (1%) | 1 | 1 | 1 | 1 | 1 | 1 | 1.5 | 1.5 | 1.5 | 1.5 | 3 | 3 |
| | (0.1%) | 0.5 | 1 | 1.5 | 1.5 | 2 | 2.5 | 3 | 3.5 | 3.5 | 3.5 | 3.5 | 4 |
| Compound 38 | (1%) | 1 | 1.5 | 1.5 | 1 | 1 | 1 | 1.5 | 2 | 2 | 2 | 2 | 2 |
| | (0.1%) | 0.5 | 2 | 2 | 2 | 2.5 | 2.5 | 3 | 3 | 3.5 | 3.5 | 3.5 | 4 |
| Tolnaftate | (1%) | 1 | 1 | 1 | 1.5 | 1 | 1 | 1.5 | 2 | 2.5 | 2.5 | 3.5 | 3.5 |
| | (0.1%) | 0 | 1.5 | 1.5 | 2 | 2.5 | 3 | 3 | 3 | 3 | 3 | 3 | 3 |
| Control (No compound treated) | | 1 | 2 | 2 | 2 | 3 | 3.5 | 3.5 | 3.5 | 3.5 | 4 | 4 | 4 |

Table 4 (Results of reverse culture test)

| Drug Tested \ Concentration | 1% | 0.1% |
| --- | --- | --- |
| Compound 53 | 0/20 | 7/20 |
| Compound 35 | 0/20 | 6/20 |
| Compound 38 | 0/20 | 6/20 |
| Tolnaftate | 0/20 | 16/20 |
| Control (No compound treated) | 20/20 | |

Test Example 3

Controlling Effects on Powdery Mildew of Barley

Young barley plants (variety: Kanto No. 6, 2-leaf stage) cultivated in porcelain pots (12 cm in diameter) were inoculated by conidia of Erysiphe graminis graminis F.Sp. hordei, the causal fungus of powdery mildew. The day after inoculation, the plants were treated on a turn table by spraying solutions (200 ppm) of the present compounds by a spray gun. The treated plants were kept at 25°C for 6 days in a greenhouse and the development of lesions was assessed. The controlling effects of the compounds were calculated in comparison with the untreated plots.

The criteria for the controlling effects are:

4; 100 - 95% control
3; 94 - 80% control
2; 79 - 60% control
1; 59 - 0% control

The results are shown in Table 5.

Table 5

| Compound No. | Concentration (ppm) | Controlling effects | Compound No. | Concentration (ppm) | Controlling effects |
|---|---|---|---|---|---|
| 1 | 200 | 3 | 38 | 200 | 4 |
| 3 | " | 4 | 42 | " | 4 |
| 5 | " | 4 | 43 | " | 4 |
| 8 | " | 4 | 45 | " | 4 |
| 9 | " | 4 | 46 | " | 4 |
| 12 | " | 3 | 47 | " | 4 |
| 15 | " | 4 | 48 | " | 4 |
| 17 | " | 4 | 50 | " | 4 |
| 20 | " | 4 | 53 | " | 4 |
| 21 | " | 4 | 54 | " | 4 |
| 22 | " | 4 | 56 | " | 4 |
| 24 | " | 4 | 57 | " | 4 |
| 25 | " | 4 | 58 | " | 4 |
| 26 | " | 4 | 61 | " | 4 |
| 27 | " | 2 | 62 | " | 4 |
| 28 | " | 4 | 64 | " | 4 |
| 29 | " | 4 | 65 | " | 4 |
| 30 | " | 2 | 67 | " | 4 |
| 32 | " | 2 | 72 | " | 4 |
| 33 | " | 4 | 75 | " | 4 |
| 34 | " | 4 | 76 | " | 4 |
| 35 | " | 4 | 77 | " | 4 |
| 36 | " | 4 | 78 | " | 4 |
| 37 | " | 4 | 79 | " | 4 |

Test Example 4

Controlling Effects on seed-borne Fusarium

Cucumber seeds infested with Fusarium oxysporum f.sp. cucumerinum were soaked in solutions (200 ppm) of the present compounds for 20 h. The treated seeds were then placed on a selective medium for Fusarium spp. (Komada medium). After a week-incubation at 25°C, the mycelial growth around the treated seeds was assessed and the controlling effects were calculated in comparison with the untreated plots. The criteria for controlling effects are the same as in Test Example 3.

The results are shown in Table 6.

Table 6

| Compound No. | Concen-tration (ppm) | Control-ling effects | Compound No. | Concen-tration (ppm) | Control-ling effects |
|---|---|---|---|---|---|
| 3 | 200 | 4 | 52 | 200 | 4 |
| 4 | " | 4 | 53 | " | 4 |
| 6 | " | 4 | 54 | " | 4 |
| 7 | " | 4 | 55 | " | 4 |
| 10 | " | 4 | 56 | " | 4 |
| 11 | " | 4 | 57 | " | 4 |
| 13 | " | 4 | 58 | " | 3 |
| 16 | " | 4 | 60 | " | 4 |
| 18 | " | 4 | 61 | " | 4 |
| 20 | " | 4 | 63 | " | 4 |
| 21 | " | 4 | 64 | " | 4 |
| 24 | " | 3 | 65 | " | 4 |
| 26 | " | 2 | 66 | " | 4 |
| 29 | " | 4 | 67 | " | 2 |
| 33 | " | 4 | 68 | " | 4 |
| 35 | " | 4 | 69 | " | 4 |
| 37 | " | 4 | 70 | " | 4 |
| 39 | " | 4 | 71 | " | 4 |
| 40 | " | 4 | 73 | " | 4 |
| 44 | " | 4 | 75 | " | 4 |
| 47 | " | 4 | 76 | " | 4 |
| 49 | " | 4 | 77 | " | 4 |
| 50 | " | 4 | 78 | " | 4 |
| 51 | " | 4 | 79 | " | 4 |

The recipes shown below as Formulation Examples 1 to 3 are for medical purposes. For these purposes, the various adjuvants and constituents to be used should be of pharmaceutically acceptable grade. In Formulation Examples 1 to 3, all parts are by weight.

Formulation Example 1

| | |
|---|---|
| Compound 35 | 1 part |
| Polyethylene glycol 300 | 95 parts |

These ingredients are mixed to prepare an endermic solution.

Formulation Example 2

| | |
|---|---|
| Compound 53 | 2 parts |
| Polyethylene glycol 400 | 40 parts |
| Polyethylene glycol 1500 | 58 parts |

These ingredients are mixed with heating to obtain a solution, which is then colled to prepare an ointment.

Formulation Example 3

| | |
|---|---|
| Copmpound 38 | 2 parts |
| 1,2-Propanediol | 5 parts |
| Glycerol stearate | 5 parts |
| Spermaceti | 5 parts |
| Isopropyl myristate | 10 parts |
| Polysorbate 60 | 4 parts |

A mixture of these compounds is heated and then cooled, after which 69 parts of water is added with stirring to prepare a cream.

In addition to the pharmaceutical formulations described above, a preparation in pharmaceutically usable forms such as injections or tablets is possible.

The recipes shown in Formulation Examples 4 to 7 are for agricultural chemicals. In these examples, all parts are by weight as in Formulation Examples 1 to 3.

Formulation Example 4

| Wettable powder | |
|---|---|
| Compound 12 | 50 parts |
| Mixture of diatomaceous earth and clay | 45 parts |
| Polyoxyethylene nonylphenyl ether | 5 parts |

These ingredients are homogeneously mixed and then pulverized to prepare a wettable powder.

Formulation Example 5

| Emulsifiable concentrate | |
|---|---|
| Compound 23 | 20 parts |
| Tetrahydrofuran | 20 parts |
| Xylene | 45 parts |
| Mixture of polyoxyethylene nonylphenyl ether and alkyl benzenesulfonate | 15 parts |

These ingredients are homogeneously mixed to prepare an emulsifiable concentrate.

Formulation Example 6

| Dust | |
|---|---|
| Compound 29 | 4 parts |
| Mixture of diatomaceous earth, clay and talc | 95 parts |
| Calcium stearate | 1 part |

These ingredients are homogeneously mixed and then pulverized to prepare a dust.

Formulation Example 7

| Granules | |
|---|---|
| Compound 46 | 3 parts |
| Mixture of bentonite and clay | 92 parts |
| Calcium lignin sulfonate | 5 parts |

These ingredients are homogeneously mixed and then pulverized, after which an adequate amount of water is added, and the resulting mixture is sufficiently kneaded and then granulated to prepare granules.

**Claims**

**Claims for the following Contracting States : BE, CH, DE, FR, GB, IT, LI, LU, NL, SE**

**1.** A ketene S,S-acetal derivative represented by the general formula (I):

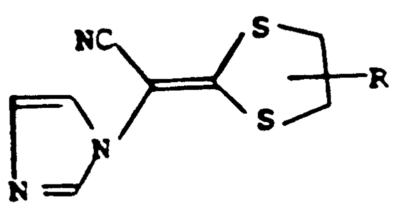

(I)

wherein R represents a hydrogen atom; an alkyl group having 1 to 8 carbon atoms; a cycloalkyl group having 3 to 6 carbon atoms; a methylene group; a lower alkenyl group; a lower alkyl group substituted by a halogen atom, a cyano group, a lower alkoxyl group, a lower alkythio group, a carbamoyl group, an acyl group, or an alkenoyloxy group; a phenyl group represented by

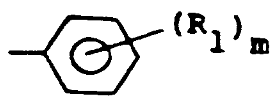

(in which $R_1$ represents a hydrogen atom, a halogen atom, a straight or branched chain lower alkyl group, a lower alkoxyl group which may be substituted by one or more halogen atoms, a phenoxy group or a methylenedioxy group, and m represents an integer of 1 to 3); a benzyl group; a methylenedioxybenzyl group; a phenoxyalkyl group; a phenoxyalkyl group substituted by a halogen atom; a naphthyl group; or a substituted or unsubstituted pyridyl group.

**2.** A ketene S,S-acetal derivative according to claim 1, wherein R represents

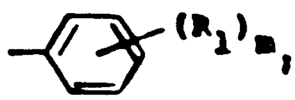

in which $R_1$ represents a halogen atom or methyl group, and m represents an integer of 1 to 3.

**3.** A ketene S,S-acetal derivative according to claim 1, which is 2-(1-imidazolyl)-2-[4-(2,4-dichlorophenyl)-1,3-dithiolan-2-ylidene]acetonitrile (E isomer).

**4.** A ketene S,S-acetal derivative according to claim 1, which is 2-(1-imidazolyl)-2-[4-(2-chloro-phenyl)-1,3-dithiolan-2-ylidene]acetonitrile (E isomer).

**5.** A ketene S,S-acetal derivative according to claim 1, which is 2-(1-imidazolyl)-2-[4-(2-bromo-phenyl)-1,3-dithiolan-2-ylidene]acetonitrile (E isomer).

**6.** A ketene S,S-acetal derivative according to claim 1, which is 2-(1-imidazolyl)-2-[4-(4-chloro-phenyl)-1,3-dithiolan-2-ylidene]acetonitrile (E isomer).

**7.** A process for producing a ketene S,S-acetal derivative according to any of claims 1 to 6, which comprises reacting 1-cyanomethylimidazole represented by the general formula (II):

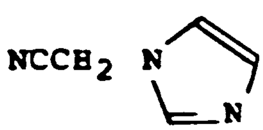

(II)

with carbon disulfide in the presence of a base, and then reacting the reaction product with a compound represented by the general formula (III):

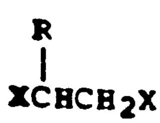

(III)

wherein R is as defined in claim 1 and X represents a halogen atom, a mesyloxy or a tosyloxy group.

**8.** An agricultural and horticultural fungicidal composition comprising a fungicidically effective amount of a ketene S,S-acetal derivative according to any of claims 1 to 6.

**9.** A pharmaceutical composition comprising a pharmaceutically effective amount of a ketene S,S-acetal derivative according to any of claims 1 to 6.

**Claims for the following Contracting State : AT**

**1.** A process for producing a ketene S,S-acetal derivative represented by the general formula (I):

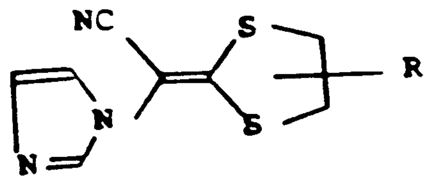

(I)

wherein $R_1$ represents a hydrogen atom; an alkyl group having 1 to 8 carbon atoms, a cycloalkyl group having 3 to 6 carbon atoms, a methylene group; a lower alkenyl group; a lower alkyl group substituted by a halogen atom, a cyano group, a lower alkoxyl group, a lower alkylthio group, a carbamoyl group, an acyl group, or an alkenoyloxy group; a phenyl group represented by

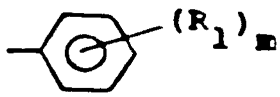

(in which $R_1$ represents a hydrogen atom, a halogen atom, a straight or branched chain lower alkyl group, a lower alkoxyl group which may be substituted by one or more halogen atoms, a phenoxy group or a methylenedioxy group, and m represents an integer of 1 to 3): a benzyl group; a methylenedioxybenzyl group; a phenoxyalkyl group; a phenoxyalkyl group substituted by a halogen atom; a naphthyl group; or a substituted or unsubstituted pyridyl group, which comprises reacting 1-cyanomethylimidazole represented by the general formula (II):

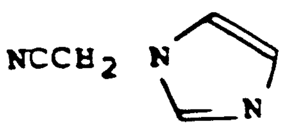

(II)

with carbon disulfide in the presence of a base, and then reacting the reaction product with a compound represented by the general formula (III):

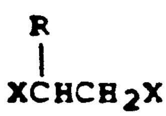

(III)

**2.** The process of claim 1 wherein R represents

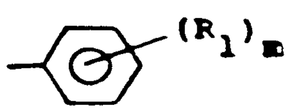

in which $R_1$ represents a halogen atom or methyl group, and m represents an integer of 1 to 3.

**3.** The process of claim 1 wherein 2-(1-imidazolyl)-2-[4-(2,4-dichlorophenyl)-1,3-dithiolan-2-ylidene]-acetonitrile (E isomer) is produced.

**4.** The process of claim 1 wherein 2-(1-imidazolyl)-2-[4-(2-chloro-phenyl)-1,3-dithiolan-2-ylidene]-acetonitrile (E isomer) is produced.

**5.** The process of claim 1 wherein 2-(1-imidazolyl)-2-[4-(2-bromo-phenyl)-1,3-dithiolan-2-ylidene]-acetonitrile (E isomer ) is produced.

**6.** The process of claim 1 wherein 2-(1-imidazolyl)-2-[4-(4-chloro-phenyl)-1,3-dithiolan-2-ylidene]-acetonitrile (E isomer) is produced.

**7.** A process for preparing an agricultural and horticultural fungicidal composition comprising mixing a fungicidically effective amount of a ketene S,S-acetal derivative produced according to any of claims 1 to 6 with usual carriers and/or diluents.

**8.** A process for preparing a pharmaceutical composition comprising mixing a pharmaceutically effective amount of a ketene S,S-acetal derivative produced according to any of claims 1 to 6 with usual carriers and/or diluents.

**Patentansprüche**

**Patentansprüche für folgende Vertragsstaaten : BE, CH, DE, FR, GB, IT, LI, LU, NL, SE**

**1.** Keten-S,S-Acetalderivat, dargestellt durch die allgemeine Formel (I):

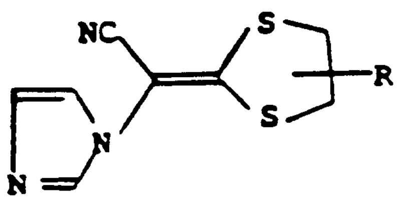

(I)

worin R ein Wasserstoffatom; eine Alkylgruppe mit 1 bis 8 Kohlenstoffatomen; eine Cycloalkylgruppe mit 3 bis 6 Kohlenstoffatomen; eine Methylengruppe; eine Niedrigalkenylgruppe; eine Niedrigalkylgruppe, substituiert durch ein Halogenatom, eine Cyanogruppe, eine Niedrigalkoxylgruppe, eine Niedrigalkylthiogruppe, eine Carbamoylgruppe, eine Acylgruppe oder eine Alkenoyloxygruppe; eine Phenylgruppe, dargestellt durch

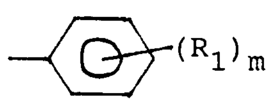

(worin $R_1$ ein Wasserstoffatom, ein Halogenatom, eine geradkettige oder verzweigte Niedrigalkylgruppe, eine Niedrigalkoxylgruppe, die substituiert sein kann durch ein oder mehrere Halogenatome, eine Phenoxygruppe oder eine Methylendioxygruppe bedeutet und m eine ganze Zahl von 1 bis 3 bedeutet); eine Benzylgruppe; eine Methylendioxybenzylgruppe; eine Phenoxyalkylgruppe; eine Phenoxyalkylgruppe, substituiert durch ein Halogenatom; eine Naphthylgruppe; oder eine substituierte oder unsubstituierte Pyridylgruppe bedeutet.

**2.** Keten-S,S-Acetalderivat nach Anspruch 1, worin R

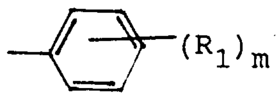

bedeutet,
worin $R_1$ ein Halogenatom oder eine Methylgruppe bedeutet und m eine ganze Zahl von 1 bis 3 bedeutet.

**3.** Keten-S,S-Acetalderivat nach Anspruch 1, welches 2-(1-Imidazolyl)-2-[4-(2,4-dichlorpheny1)-1,3-dithiolan-2-yliden]acetonitril (E-Isomer) ist.

**4.** Keten-S,S-Acetalderivat nach Anspruch 1, welches 2-(1-Imidazolyl)-2-[4-(2-chlorphenyl)-1,3-dithiolan-2-yliden]acetonitril (E-Isomer) ist.

**5.** Keten-S,S-Acetalderivat nach Anspruch 1, welches 2-(1-Imidazolyl)-2-[4-(2-bromphenyl)-1,3-dithiolan-2-yliden]acetonitril (E-Isomer) ist.

**6.** Keten-S,S-Acetalderivat nach Anspruch 1, welches 2-(1-Imidazolyl)-2-[4-(4-chlorphenyl)-1,3-dithiolan-2-yliden]acetonitril (E-Isomer) ist.

**7.** Verfahren zur Herstellung eines Keten-S,S-Acetalderivats nach einem der Ansprüche 1 bis 6, das die Umsetzung von 1-Cyanomethylimidazol, dargestellt durch die allgemeine Formel (II):

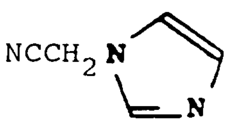

(II)

mit Kohlenstoffdisulfid in Gegenwart einer Base und die anschließende Umsetzung des Reaktionsprodukts mit einer Verbindung, dargestellt durch die allgemeine Formel (III):

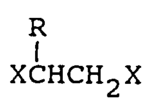

(III)

worin R wie in Anspruch 1 definiert ist und X ein Halogenatom, eine Mesyloxy- oder eine Tosyloxygruppe bedeutet, umfaßt.

**8.** Landwirtschaftliche und gartenbauliche fungizide Zusammensetzung, umfassend eine fungizid wirksame Menge eines Keten-S,S-Acetalderivats nach einem der Ansprüche 1 bis 6.

**9.** Pharmazeutische Zusammensetzung, umfassend eine pharmazeutisch wirksame Menge eines Keten-S,S-Acetalderivats nach einem der Ansprüche 1 bis 6.

**Patentansprüche für folgenden Vertragsstaat : AT**

1. Verfahren zur Herstellung eines Keten-S,S,-Acetalderivats, dargestellt durch die allgemeine Formel (I):

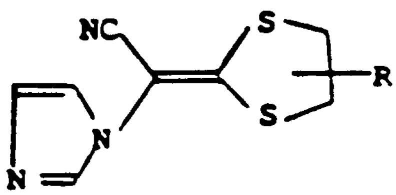

(I)

worin R ein Wasserstoffatom; eine Alkylgruppe mit 1 bis 8 Kohlenstoffatomen; eine Cycloalkylgruppe mit 3 bis 6 Kohlenstoffatomen; eine Methylengruppe; eine Niedrigalkenylgruppe; eine Niedrigalkylgruppe, substituiert durch ein Halogenatom, eine Cyanogruppe, eine Niedrigalkoxylgruppe, eine Niedrigalkylthiogruppe, eine Carbamoylgruppe, eine Acylgruppe oder eine Alkenoyloxygruppe; eine Phenylgruppe, dargestellt durch

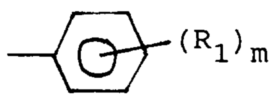

(worin $R_1$ ein Wasserstoffatom, ein Halogenatom, eine geradkettige oder verzweigte Niedrigalkylgruppe, eine Niedrigalkoxylgruppe, die substituiert sein kann durch ein oder mehrere Halogenatome, eine Phenoxygruppe oder eine Methylendioxygruppe bedeutet und m eine ganze Zahl von 1 bis 3 bedeutet); eine Benzylgruppe; eine Methylendioxybenzylgruppe; eine Phenoxyalkylgruppe; eine Phenoxyalkylgruppe, substituiert durch ein Halogenatom; eine Naphthylgruppe; oder eine substituierte oder unsubstituierte Pyridylgruppe bedeutet, das die Umsetzung von 1-Cyanomethylimidazol, dargestellt durch die allgemeine Formel (II):

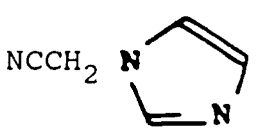

(II)

mit Kohlenstoffdisulfid in Gegenwart einer Base und die anschließende Umsetzung des Reaktionsprodukts mit einer Verbindung, dargestellt durch die allgemeine Formel (III):

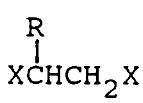

(III)

worin R wie in Anspruch 1 definiert ist und X ein Halogenatom, eine Mesyloxy- oder eine Tosyloxygruppe bedeutet, umfaßt.

2. Verfahren nach Anspruch 1, worin R

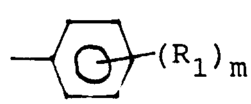

bedeutet, worin $R_1$ ein Halogenatom oder eine Methylgruppe bedeutet und m eine ganze Zahl von 1 bis 3 bedeutet.

**3.** Verfahren nach Anspruch 1, worin 2-(1-Imidazolyl)-2-[4-(2,4-dichlorphenyl)-1,3-dithiolan-2-yliden]-acetonitril (E-Isomer) hergestellt wird.

**4.** Verfahren nach Anspruch 1, worin 2-(1-Imidazolyl)-2-[4-(2-chlorphenyl)-1,3-dithiolan-2-yliden]acetonitril (E-Isomer) hergestellt wird.

**5.** Verfahren nach Anspruch 1, worin 2-(1-Imidazolyl)-2-[4-(2-bromphenyl)-1,3-dithiolan-2-yliden]acetonitril (E-Isomer) hergestellt wird.

**6.** Verfahren nach Anspruch 1, worin 2-(1-Imidazolyl)-2-[4-(4-chlorphenyl)-1,3- dithiolan-2-yliden]-acetonitril (E-Isomer) hergestellt wird.

**7.** Verfahren zur Herstellung einer landwirtschaftlichen und gartenbaulichen fungiziden Zusammensetzung, umfassend das Mischen einer fungizid wirksamen Menge eines Keten-S,S-Acetalderivats, hergestellt nach einem der Ansprüche 1 bis 6, mit üblichen Trägern und/oder Verdünnungsmitteln.

**8.** Verfahren zur Herstellung einer pharmazeutischen Zusammensetzung, umfassend das Mischen einer pharmazeutisch wirksamen Menge eines Keten-S,S-Acetalderivats, hergestellt nach einem der Ansprüche 1 bis 6, mit üblichen Trägern und/oder Verdünnungsmitteln.

**Revendications**
**Revendications pour les Etats contractants suivants : BE, CH, DE, FR, GB, IT, LI, LU, NL, SE**

**1.** S,S-acétal de cétène représenté par la formule générale (I) :

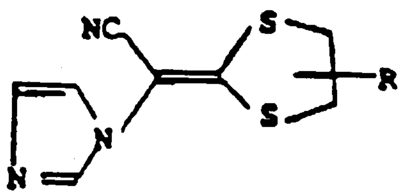

(I)

dans laquelle R représente un atome d'hydrogène; un groupe alkyle en C1 à C8; un groupe cycloalkyle en C3 à C6; un groupe méthylène; un groupe alcényle inférieur; un groupe alkyle inférieur substitué par un atome d'halogène, un groupe cyano, un groupe alcoxy inférieur, un groupe alkylthio inférieur, un groupe carbamoyle, un groupe acyle ou un groupe alcénoyloxy; un groupe phényle représenté par la formule

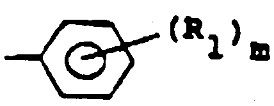

(dans laquelle $R_1$ représente un atome d'hydrogène, un atome d'halogène, un groupe alkyle inférieur à chaîne linéaire ou ramifiée, un groupe alcoxy inférieur qui peut être substitué par un ou plusieurs atomes d'halogène, un groupe phénoxy ou un groupe methylènedioxy, et m représente un entier de 1 à 3); un groupe benzyle; un groupe méthylènedioxybenzyle ; un groupe phénoxyalkyle ; un groupe phénoxyalkyle substitué par un atome d'halogène ; un groupe naphtyle ; ou un groupe pyridyle substitué ou non substitué.

2. S,S-acétal de cétène selon la revendication 1, dans lequel R répond à la formule

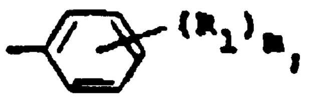

dans laquelle $R_1$ représente un atome d'halogène ou un groupe méthyle, et m représente un entier de 1 à 3.

3. S,S-acétal de cétène selon la revendication 1, qui est le 2-(1-imidazolyl)-2-[4-(2,4-dichlorophényl)-1,3-ditpiolan-2-ylidène]acétonitrile (isomère E).

4. S,S- acétal de cétène selon la revendication 1, qui est le 2-(1-imidazolyl)-2-[4-(2-chloro-phényl)-1,3-dithiolan-2-ylidène] acétonitrile (isomère E).

5. S,S- acétal de cétène selon la revendication 1, qui est le 2-(1-imidazolyl)-2-[4-(2-bromo-phényl)- 1,3-dithiolan-2-ylidène] acétonitrile (isomère E).

6. S,S- acétal de cétène selon la revendication 1, qui est le 2-(1-imidazolyl)-2-[4-(4-chloro-phényl)-1,3-dithiolan-2-ylidène] acétonitrile (isomère E).

7. Procédé de préparation d'un S,S-acétal de cétène selon l'une quelconque des revendications 1 à 6, qui comprend le fait de faire réagir un 1-cyanométhylimidazole représenté par la formule générale (II) :

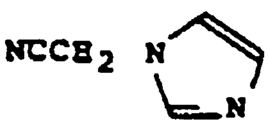

(II)

avec du sulfure de carbone en présence d'une base, puis le fait de faire réagir le produit de la réaction avec un composé représenté par la formule générale (III):

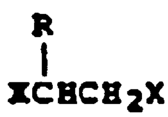

(III)

dans laquelle R est tel que défini dans la revendication 1 et X représente un atome d'halogène, un groupe méthyloxy ou un groupe tosyloxy.

8. Composition fongicide pour l'agriculture et l'horticulture comprenant un quantité efficace comme fongicide d'un S,S-acétal de cétène selon l'une quelconque des revendications 1 à 6.

9. Composition pharmaceutique comprenant une quantité pharmaceutiquement efficace d'un S,S-acétal de cétène selon l'une quelconque des revendications 1 à 6.

**Revendications pour l'Etat contractant suivant : AT**

**1.** Procédé de préparation d'un S,S-acétal de cétène représenté par la formule générale (I):

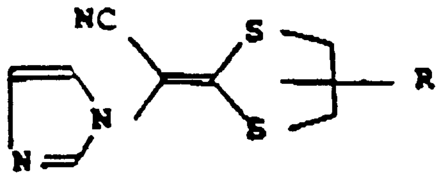

(I)

dans laquelle R représente un atome d'hydrogène; un groupe alkyle en C1 à C8, un groupe cycloalkyle en C3 à C6, un groupe méthylène; un groupe alcényle inférieur; un groupe alkyle inférieur substitué par un atome d' halogène, un groupe cyano, un groupe alcoxy inférieur, un groupe alkylthio inférieur, un groupe carbamoyle, un groupe acyle ou un groupe alcénoyloxy; un groupe phényle représenté par la formule

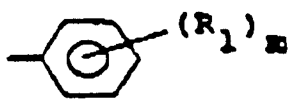

(dans laquelle $R_1$ représente un atome d'hydrogène, un atome d'halogène, un groupe alkyle inférieur à chaîne linéraire ou ramifiée, un groupe alcoxy inférieur qui peut être substitué par un ou plusieurs atome d'halogène, un groupe phénoxy ou un groupe méthylènedioxy, et m représente un entier de 1 à 3); un groupe benzyle; un groupe méthylènedioxybenzyle; un groupe phénoxyalkyle, un groupe phénoxyalkyle substitué par un atome d'halogène; un groupe naphtyle; ou un groupe pyridyle substitué ou non substitué, qui comprend le fait de faire réagir le 1-cyanométhylimidazole représenté par la formule générale (II):

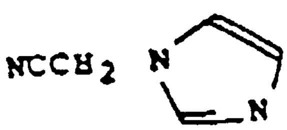

(II)

avec du sulfure de carbone en présence d'une base, puis de faire réagir le produit de la réaction avec un composé représenté par la formule (III):

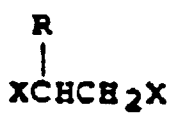

(III)

**2.** Procédé selon la revendication 1, dans lequel R répond à la formule

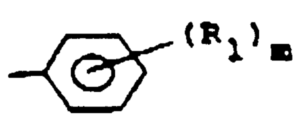

dans laquelle $R_1$ représente un atome d'halogène ou un groupe méthyle et m représente un entier

de 1 à 3.

3. Procédé selon la revendication 1, dans lequel on prépare le 2-(1-imidazolyl)-2-[4-(2,4-dichlorophényl)-1,3-dithiolan-2-ylidène] acétonitrile (isomère E).

4. Procédé selon la revendication 1, dans lequel on prépare le 2-(1-imidazolyl)-2-[4-(2,4-chloro-phényl)-1,3-dithiolan-2-ylidène] acétonitrile (isomère E).

5. Procédé selon la revendication 1, dans lequel on prépare le 2-(1-imidazolyl)-2-[4-(2-bromo-phényl)- 1,3-dithiolan-2-ylidène] acétonitrile (isomère E).

6. Procédé selon la revendication 1, dans lequel on prépare le 2-(1-imidazolyl)-2-[4-( 4-chloro-phényl)-1,3-dithialan-2-ylidène] acétonitrile (isomère E).

7. Procédé de préparation d'une composition fongicide pour l'agriculture et l'horticulture comprenant le mélange d'une quantité efficace comme fongicide d'un S,S-acétal de cétène préparé selon l'une quelconque des revendications 1 à 6 avec des supports et/ou diluants usuels.

8. Procédé de préparation d'une composition pharmaceutique comprenant le mélange d'une quantité pharmaceutiquement efficace d'un S,S-acétal de cétène préparé selon l'une quelconque des revendications 1 à 6 avec des supports et/ou diluants usuels.